Europäisches Patentamt

European Patent Office

Office européen des brevets

(11) Numéro de publication: **0 315 503 B2**

(12)

# NOUVEAU FASCICULE DE BREVET EUROPEEN

(45) Date de publication de nouveau fascicule du brevet:
**08.11.95**

(51) Int. Cl.⁶: **A61K 7/16**, C01B 33/193, C01B 33/12

(21) Numéro de dépôt: 88402652.7

(22) Date de dépôt: **21.10.88**

(54) **Silice pour compositions dentifrices compatible notamment avec la chlorhexidine.**

(30) Priorité: **04.11.87 FR 8715275**

(43) Date de publication de la demande:
**10.05.89 Bulletin 89/19**

(45) Mention de la délivrance du brevet:
**08.04.92 Bulletin 92/15**

(45) Mention de la décision
concernant l'opposition:
**08.11.95 Bulletin 95/45**

(84) Etats contractants désignés:
**AT BE CH DE ES FR GB GR IT LI LU NL SE**

(56) Documents cités:
**EP-B- 0 031 271      EP-B- 0 078 909
EP-B- 0 097 476      DE-A- 1 667 701
DE-A- 2 628 975      US-A- 4 036 949
US-A- 4 340 583      US-A- 4 562 066**

(73) Titulaire: **RHONE-POULENC CHIMIE
25, Ouai Paul Doumer
F-92408 Courbevoie Cédex (FR)**

(72) Inventeur: **Persello, Jacques
14 Chemin de Calisse
310 La Boisse
F-01120 Montluel (FR)**

(74) Mandataire: **Dubruc, Philippe et al
RHONE-POULENC CHIMIE,
Direction de la Propriété Industrielle,
25, Ouai Paul Doumer
F-92408 Courbevoie Cédex (FR)**

EP 0 315 503 B2

**Description**

La présente invention concerne une silice utilisable notamment dans les compositions dentifrices.

On sait que la silice est couramment utilisée dans la préparation de compositions dentifrices. Elle peut y jouer d'ailleurs plusieurs rôles.

Elle agit tout d'abord comme agent abrasif en aidant par son action mécanique à l'élimination de la plaque dentaire.

Elle peut aussi jouer le rôle d'agent épaississant pour conférer des propriétés rhéologiques déterminées au dentifrice ainsi que d'agent optique pour lui donner la coloration souhaitée.

Par ailleurs, on sait que les dentifrices contiennent des agents divers notamment pour la prévention des caries, pour diminuer la formation de la plaque dentaire ou le dépôt de tartre sur les dents. Parmi ces agents on peut citer en particulier les fluorures. D'autres éléments sont aussi utilisés tels les phosphates, les pyrophosphates, les polyphosphates, les polyphosphonates, les guanidines notamment les bis-biguanides dont l'un des éléments le plus employé est la chlorhexidine. Les formulations dentifrices peuvent aussi comporter du zinc, des aromes, des parfums. etc...

La présence de ces agents dans le dentifrice, pose le problème de leur compatibilité avec la silice. En effect à cause notamment de ses capacités adsorbantes, celle-ci peut avoir tendance à réagir avec ces agents de telle sorte qu'ils ne soient plus disponibles pour exercer les effets thérapeutiques décrits plus haut.

L'objet de l'invention est donc de trouver des silices compatibles avec les agents mentionnés ci-dessus notamment les guanidines et donc parfaitement utilisables dans la formulation de dentifrices.

Or, à cet effet, la Demanderesse s'est aperçu que les propriétés de compatibilité recherchées dépendaient essentiellement de la chimie de surface de la silice utilisée. La Demanderesse a ainsi établi un certain nombre de conditions sur la surface des silices pour que celles-ci soient compatibles.

La silice selon l'invention est caractérisée en ce qu'elle présente une compatibilité avec les produits du type guanidines et en particulier la chlorhexidine d'au moins 65 % et plus particulièrement d'au moins 90 %.

Par ailleurs, la silice selon l'invention, compatible notammment avec les produits du type guanidines et en particulier la chlorhexidine, est aussi caractérisée en ce qu'elle présente une chimie de surface telle que sa fonction d'acidité Ho soit d'au moins 3,3.

D'autre part, le procédé de préparation de la silice du type décrit ci-dessus comprend selon une première variante une réaction d'un silicate avec un acide, ce par quoi on obtient une suspension ou un gel de silice, une séparation et un séchage de la silice et il est caractérisé en ce qu'après la séparation de la silice, on effectue un lavage à l'eau du gâteau résultant de cette séparation jusqu'à ce que la conductivité du filtrat soit d'au plus 200 microsiemens cm$^{-1}$.

Selon une seconde variante, le procédé de préparation d'une silice de l'invention comprend une réaction d'un silicate avec un acide ce par quoi on obtient une suspension ou un gel de silice, une séparation et un séchage de la silice et il est caractérisé en ce qu'on effectue un premier lavage à l'eau du gâteau résultant de cette séparation puis un second lavage ou traitement avec une solution acide.

D'autres caractéristiques et avantages de l'invention seront mieux compris à la lecture de la description et des exemples concrets qui vont suivre.

Comme cela été indiqué en introduction, les caractéristiques essentielles des silices de l'invention résident dans leur chimie de surface. Plus précisément, un des aspects à prendre en compte dans cette chimie de surface est l'acidité. A ce sujet une des caractéristiques des silices de l'invention est la force de leurs sites acides de surface.

Ici l'acidité est pris dans le sens de Lewis, c'est-a-dire qu'elle traduit la tendance d'un site à accepter une paire d'électrons d'une base selon l'équilibre :

$$B : + A = BA$$

On utilise pour caractériser les silices de l'invention la notion de "fonction d'acidité" Ho, développée par Hammett, pour mesurer la tendance de l'acide, la silice dans le cas présent, à accepter une paire d'électrons d'une base.

La fonction Ho est ainsi définie par la relation classique :

$$\frac{pKa + \log\ (B:)}{(B:)(A)} = Ho$$

Pour déterminer la force des sites acides d'une silice de l'invention par la méthode de Hammett on utilise la méthode des indicateurs décrite à l'origine par Walling (J. Am. chem. Soc. 1950, 72, 1164). La force des sites acides est déterminée par des indicateurs colorés dont on connaît, dans les conditions de l'emploi, le pKa de passage entre les formes acides et basiques.

Ainsi, plus bas est le pKa de l'indicateur subissant le changement de coloration, et plus forte est l'acidité du site. On a rassemblé dans le tableau ci-dessous, à titre d'exemple une liste d'indicateurs de Hammett susceptibles d'être utilisés pour encadrer la valeur de Ho en déterminant sous quelle forme sont adsorbés deux indicateurs successifs.

| Indicateur | couleur forme basique | forme acide | pKa |
|---|---|---|---|
| Neutral red | jaune | rouge | + 6,8 |
| Methyl red | jaune | rouge | + 4,8 |
| Phenylazonaphthylamine | jaune | rouge | + 4,0 |
| p-Dimethylaminoazobenzene | jaune | rouge | + 3,3 |
| 2-Amino-5-azotoluene | jaune | rouge | + 2,O |
| Benzeneazodiphenylamine | jaune | rouge | + 1,5 |
| 4-Dimethylaminoazo-1-naphtalene | jaune | rouge | + 1,2 |
| Crystal violet | bleu | jaune | + O,8 |
| p-Nitrobenzeneazo-(p'-nitro)diphenylamine | orange | violet | + 0,43 |
| Dicinnamalacetone | jaune | rouge | - 3,0 |
| Benzalacetophenone | incolore | jaune | - 5,6 |
| Anthraquinone | incolore | jaune | - 8,2 |

La couleur des indicateurs adsorbés sur une silice est une mesure de la force des sites acides. Si la couleur est celle de la forme acide de l'indicateur, alors a valeur de la fonction Ho de la surface est égale ou inférieure au pKa de l'indicateur.

Des valeurs faibles de Ho, correspondent à des sites acides de force élevées.

Ainsi, par exemple une silice donnant une coloration rouge avec le p-Dimethylaminobenzene et jaune avec le 2-Amino-5-azotoluene aura une fonction d'acidité Ho comprise entre 3,3 et 2.

Expérimentalement, le dosage s'effectue avec 0,2 g de silice placée dans un tube à essai en présence d'une solution d'indicateur à 100 mg/l dans le cyclohexane.

La silice est au préalable séchée à 190°C pendant 2 heures et conservée à l'abri de l'humidité dans un dessicateur.

Par agitation, l'adsorption, si elle a lieu, se produit en quelques minutes et on note le changement de couleur visible à l'oeil nu ou éventuellement en étudiant les spectres d'absorption caractéristiques des indicateurs colorés adsorbés, aussi bien dans leur forme acide que dans leur forme basique.

La première caractéristique des silices de l'invention est qu'elles présentent une fonction d'acidité telle que déterminée ci-dessus d'au moins 3,3.

La force et la nature des sites acides de surface peut aussi être mesurée par spectrométrie infra-rouge de la pyridine adsorbée sur la silice.

On sait que la quantité de pyridine adsorbée sur un solide permet de déterminer notamment la nature des sites acides de surface.

La pyridine est une base relativement forte (pKb = 9) et de fait ne réagit pas avec les sites faibles contrairement à NH3 (pKb = 5).

La formation d'on pyridinium (PyH+) permettra en plus, de différentier les sites de type Lewis et de Bronsted.

Des informations sur l'acidité d'une surface d'un solide peuvent aussi être obtenues en étudiant les bandes d'absorption de la pyridine dans le domaine 1700 cm-1 à 1400 cm-1.

De plus la valeur du décalage des bandes caractéristique de la pyridine et de ses formes ionisées, avant et après adsorption permet de quantifier la force des sites acides.

L'ion pyridinium donne une bande à 1540 cm-1 alors que la pyridine liée par liaisons H ou de coordination donne des bandes dans la région 1440-1465 cm-1. De plus, il apparaît que la bande de la pyridine située à 1583 cm-1 est déplacée quand la pyridine est adsorbée. Cette bande indique la présence de sites acides de Lewis. La force acide de ces derniers étant proportionnelle au déplacement de la bande.

En résumé, il est possible d'utiliser les bandes à 1540 , 1640 et 1485 cm-1 pour définir l'acidité de type Bronsted et les bandes situées dans la région 1440-1465 cm-1 pour l'acidité de Lewis.

Expérimentalement, les mesures sont effectuées sur une suspension de silice dans le tétrachlorure de carbone en présence de pyridine.

La silice est séchée au préalable à 190°C pendant 2 heures et conservée à l'abri de l'humidité. Après refroidissement on disperse par agitation magnétique suivie d'une dispersion par ultra-sons (10 mn), 1 g de silice dans 50 ml de CC14.

On ajoute 0,8 mg de pyridine par mètre carré de silice introduite. On chauffe à reflux sous agitation pendant 1 heure.

Le même protocole est utilisé pour préparer une solution témoin de même concentration en pyridine mais sans silice et une suspension témoin de même concentration en silice mais en absence de pyridine.

Le spectre d'adsorption de la pyridine est réalisé par spectroscopie infra-rouge sur la suspension, la solution de pyridine en absence de silice et la suspension de silice sans ajout de pyridine.

On soustrait sur le spectre obtenu à partir de la suspension le spectre correspondant à la solution témoin et le spectre correspondant à la suspension témoin.

La silice sera caractérisée par la position des bandes restantes et le déplacement des bandes d'absorption de la pyridine et de l'ion pyridinium par rapport à la position des bandes de leurs formes non adsorbées.

D'une manière générale le spectre obtenu ne doit pas présenter de pic pyridinium (bande à 1540 cm$^{-1}$) l'absence de ce pic indiquant bien que la silice présente une fonction acide Ho d'au moins 3,3.

L'importance du déplacement des bandes pyridine et pyridine adsorbée permet d'apprécier l'acidité plus ou moins grande des sites acides de surface. D'une manière générale pour la bande de 1440cm$^{-1}$ ce déplacement ( ) doit être d'au plus 10 cm$^{-1}$ plus particulièrement d'au plus 5 cm$^{-1}$.

Selon le mode préféré de l'invention, ce déplacement ( ) est nul.

La silice telle que définie ci-dessus présente une bonne compatibilité avec la chlorhexidine, cette compatiblité, mesurée par le test décrit ci-après pouvant être d'au moins 65 % notamment d'au moins 80 % et préférentiellement d'au moins 90 %.

Toutefois, selon un mode de réalisation particulier de l'invention, les silices peuvent être compatibles en outre avec le fluor. Dans ce cas, elles présentent une teneur en anions du type $SO_4^{2-}$, $Cl^-$, $NO_3^-$, $PO_4^{3-}$, $CO_3^{2-}$ d'au plus $5.10^{-3}$ moles pour 100 g de silice.

Cette compatibilité sera d'autant plus grande que cette teneur sera faible. Selon des variantes préférées elle sera d'au plus $1.10^{-3}$ moles, et plus particulièrement $0,2.10^{-3}$ moles pour 100 g de silice.

Dans le cas de silices préparées à partir d'acide sulfurique, on exprimera plus commodément cette teneur en anion par une teneur exprimée en $SO_4$ et en poids. Dans ce cas cette teneur est d'au plus 0,5 %.

Selon une variante préférée de l'invention cette teneur est d'au plus 0,1 % et plus particulièrement d'au plus 0,02 %.

Cette compatibilité peut encore être améliorée, notamment vis-à-vis de certains éléments comme le zinc si l'on observe des conditions sur le nombre de sites acides de surface. Ce nombre peut se mesurer en nombre de groupements OH ou silanols par nm2.

La détermination de ce nombre se fait de a manière suivante :

Le nombre de sites OH de surface est assimilé à la quantité d'eau libérée par la silice entre 190°C et 900°C.

Les échantillons de silice sont préalablement séchés à 105°C pendant 2 heures.

Une masse Po de silice est placée dans une thermobalance et est portée à 190°C pendant 2 heures; soit P190 la masse obtenue. La silice est portée ensuite à 900°C pendant 2 heures, soit P900 la nouvelle masse obtenue.

Le nombre de sites OH est calculé par l'équation suivante :

$$NOH = \frac{66922.2}{A} \times \frac{P190 - P900}{P190}$$

Où NOH est le nombre de sites OH par nm2 de surface :

A est la surface spécifique du solide (BET) en m2/g. Dans le cas présent, les silices de l'invention présenteront avantageusement un nombre de OH/nm2 inférieur ou égal à 15, plus particulièrement d'au plus 12 et notamment compris entre 3 et 12.

La nature des sites OH des silices de l'invention qui constitue aussi une caractérisation de leur chimie de surface peut aussi être appréciée par le point de charge nulle.

Ce point de charge nulle (PZC) est défini par le pH d'une suspension de silice pour lequel la charge électrique de la surface du solide est nulle et cela quelle que soit la force ionique du milieu. Ce PZC mesure le pH réel de la surface, dans la mesure où celle-ci est libre de toutes impuretés de type ionique.

La charge électrique est déterminée par potentiométrie. Le principe de la méthode est basé sur le bilan global des protons adsorbés ou désorbés sur la surface de la silice à un pH donné.

A partir des équations décrivant le bilan global de l'opération, il est facile de montrer que la charge électrique C de la surface, prise par rapport à une référence correspondant à une charge de surface nulle, est donnée par l'équation :

$$C = \frac{F}{A.M} (H - OH)$$

dans laquelle :

A représente la surface spécifique du solide en m2/g,

M est la quantité de solide dans la suspension en g,

F est le Faraday,

H ou OH représente la variation par unité de surface de l'excès d'ions $H^+$ ou $OH^-$ respectivement sur le solide.

Le protocole expérimental de détermination du PZC est le suivant :

On utilise la méthode décrite par Berube et de Bruyn (J. Colloid Interface Sc. 1968, 27, 305).

La silice est lavée au préalable dans de l'eau déionisée de haute résistivité (10 Mega.Ohm.cm), séchée puis dégazée.

Pratiquement, on prépare une série de solutions à pHo : 8,5 par ajout de KOH ou $HNO_3$ et contenant un électrolyte indifférent ($KHO_3$) à une concentration variable entre $10^{-5}$ et $10^{-1}$ Mole/l.

A ces solutions on ajoute une masse donnée de silice et on laisse le pH des suspensions obtenues se stabiliser sous agitation, à 25°C et sous azote pendant 24 heures ; soit pH'o sa valeur.

Des solutions étalons sont constituées par le surnagenat obtenu par centrifugation pendant 30 min à 1000t/min d'une partie de ces mêmes suspensions : soit pH'o le pH de ces surnageants.

On ramène ensuite le pH d'un volume connu de ses suspensions et des solutions étalons correspondantes, à pHo en rajoutant la quantité nécessaire de KOH et on laisse les suspensions et les solutions étalons se stabiliser pendant 4 heures.

Soit Voh.Noh le nombre d'équivalents ce base ajoutée pour passer de pH'o à pHo d'un volume (V) connu de suspension ou de solution étalon.

Le dosage potentiométrique des suspensions et des solutions étalons est effectué à partir de pHo par addition d'acide nitrique jusqu'à pHf = 2,0.

Préférentiellement on procède par addition d'incrément d'acide correspondant à une variation de pH de 0,2 unité de pH. Après chaque addition, le pH est stabilisé pendant 1 min.

Soit Vh.Nh le nombre d'équivalents d'acide pour parvenir à pHf.

A partir de pHo, on trace le terme (Vh.Nh - Voh.Noh) en fonction des pH incrémentés pour toutes les suspensions (3 forces ioniques au moins) et pour toutes les solutions étalons correspondantes.

Pour chaque valeur de pH (pas de 0,2 unité) on fait ensuite la différence entre la consommation de $H^+$ ou $OH^-$ pour la suspension et pour la solution étalon correspondante. On renouvelle cette opération pour toutes les forces ioniques.

Ceci donne le terme (H - OH) correspondant à la consommation en protons de la surface. La charge de surface est calculée par l'équation ci-dessus.

On trace ensuite les courbes charge de surface en fonction du pH pour toutes les forces ioniques considérées. Le PZC est défini par l'intersection des courbes.

On ajuste la concentration en silice en fonction de la surface spécifique de celle-ci.

Par exemple, on utilise des suspensions à 2 % pour les silices de 50 m2/g à 3 forces ioniques (0,1; 0,01 et 0,001 mole/l).

Le dosage est effectué sur 100 ml de suspension en utilisant de l'hydroxide de potassium à 0,1 M.

5

En pratique, il est préférable que la valeur de ce PZC soit d'au moins 3 plus particulièrement compris entre 4 et 6. Dans le cas d'une meilleur compatibilité avec le zinc, elle est d'au plus 6,5. Pour la compatibilité fluor, il est préférable d'avoir un PZC d'au plus 7.

Toujours pour l'amélioration de la comptabilité, notamment vis-à-vis du fluor, il est intéressant que la teneur en aluminium des silices de l'invention soit au plus de 500 ppm.

D'autre part la teneur en fer des silices de l'invention peut être avantageusement d'au plus 200 ppm.

Par ailleurs d'une manière préférentielle la teneur en calcium peut être d'au plus 500 ppm et plus particulièrement d'au plus 300 ppm.

Les silices de l'invention présentent aussi de préférence une teneur en carbone d'au plus 50 ppm et plus particulièrement d'au plus 10 ppm.

Le pH des silices selon l'invention mesuré selon la norme NFT 45-007 est généralement au plus de 8. Il est plus particulièrement compris entre 6,0 et 7,5.

Les caractéristiques ci-dessus permettent d'avoir une silice qui soit compatible au moins avec les guanidines et en particulier la chlorhexidine et suivant les cas avec en plus les fluorures, les phosphates et leurs dérivés et le zinc notamment.

Outre les caractéristique de chimie de surface qui viennent d'être décrites ci-dessus et qui conditionnent les compatibilités, les silices de l'invention présentent aussi des caractéristiques physiques qui les rendent parfaitement adaptées à leur application en dentifrice. Ces caractéristiques de type structurel vont être décrites ci-dessous.

Généralement la surface BET des silices de l'invention est comprise entre 40 et 600 m2/g, plus particulièrement entre 40 et 350 m2/g. Leur surface CTAB varie habituellement entre 40 et 400 m2/g, plus particulièrement entre 40 et 200 m2/g.

La surface BET est déterminée selon la methode de BRUNAUER-EMMET-TELLER décrite dans the Journal of the American Chemical Society vol. 60, page 309, February 1938 et selon la norme NF X11-622 (3.3).

La surface CTAB est la surface externe déterminée selon la norme ASTM D3765 mais en pratiquant l'adsorption de bromure d'hexadécyltriméthyl ammonium (CTAB) à pH 9 et en prenant comme aire projetée de la molécule de CTAB 35 A$^{\circ}$2.

Les silices de l'invention peuvent bien entendu correspondre aux trois types habituellement distingués dans le domaine du dentifrice.

Ainsi, les silices de l'invention peuvent être du type abrasif. Elles présentent alors une surface BET comprise entre 40 et 300 m2/g. Dans ce cas, la surface CTAB est comprise entre 40 et 100 m2/g.

Les silices de l'invention peuvent aussi être au type épaississant. Elles ont alors une surface BET comprise entre 120 et 450 m2/g plus particulièrement 120 et 200 m2/g. Elles pourront présenter alors une surface CTAB entre 120 et 400 m2/g, plus particulièrement entre 120 et 200 m2/g.

Enfin, selon un troisième type, les silices de l'invention peuvent être bifonctionnelles. Elles possèdent ici une surface BET comprise entre 80 et 200 m2/g. La surface CTAB est alors comprise entre 80 et 200 m2/g.

Les silices de l'invention peuvent aussi présenter une prise d'huile comprise entre 80 et 500 cm3/100 g déterminée selon la norme NPT 30-022 (mars 53) en mettant en oeuvre le phtalate de dibutyle.

Plus précisément, cette prise d'huile sera comprise entre 100 et 140 cm3/100 g pour les silices abrasives, 200 et 400 pour les silices épaississantes et 100 et 300 pour les bifonctionnelles.

Par ailleurs, toujours en vue de l'application en dentifrice, les silices ont préférentiellement une taille de particules comprise entre 1 et 10 $\mu$m.

Cette taille moyenne de particules est mesurée par Counter-Coulter.

La densité apparente variera généralement entre 0,01 et 0,3. Selon un mode de réalisation particulier de l'invention, les silices sont des silices de précipitation.

Enfin, les silices de l'invention présentent un indice de réfraction généralement compris entra 1,440 et 1,465.

Le procédé de préparation des silices de l'invention va maintenant être décrit plus particulièrement.

Comme indiqué plus haut, ce procédé est du type comprenant la réaction d'un silicate avec un acide ce qui donne lieu à la formation d'une suspension ou d'un gel de silice.

Il est à noter que l'on peut utiliser tout mode opératoire connu pour arriver à cette suspension ou ce gel, (addition d'acide sur un pied de cuve de silicate, addition simultanée totale ou partielle d'acide et de silicate sur un pied de cuve d'eau ou de solution de silicate etc...) le choix se faisant essentiellement en fonction des caractéristiques physiques de la silice que l'on désire obtenir. On notera qu'il peut être avantageux d'amener le pH de la suspension ou du gel obtenu à une valeur d'au plus 6 et plus particulièrement comprise entre 4 et 6.

On procède alors à la séparation de la silice du milieu réactionnel selon tout moyen connu, filtre sous vide ou filtre presse par exemple.

On recueille ainsi un gâteau de silice.

Ensuite le procédé peut être mise en oeuvre selon deux variantes principales.

La première variante concerne la préparation de silices compatibles essentiellement avec les guanidines et notamment la chlorhexydine.

Dans ce cas, le procédé comporte un lavage du gâteau. Ce lavage se fait à l'eau, généralement à l'eau désionisée jusqu'à ce que l'on obtienne un filtrat de lavage dont la conductivité soit d'au plus 200 microsiemens cm$^{-1}$.

Si l'on désire améliorer encore les compatibilités des silices obtenues par le procédé, ce lavage sera poursuivi à un degré plus élevé.

En particulier, selon un mode de réalisation préféré, on continuera le lavage jusqu'à une conductivité d'au plus 100 microsiemens cm$^{-1}$.

Une fois le gâteau de silice lavé selon le mode opératoire décrit plus haut celui-ci ou, s'il est délité, la suspension de délitage est séché selon tout moyen connu. Le séchage peut se faire notamment par atomisation. Le produit séché sera broyé si nécessaire pour obtenir la granulométrie désirée.

La seconde variante du procédé concerne la préparation de silices compatibles avec, outre les guanidines, d'autres éléments tels que le fluor, le zinc et les phosphates.

Dans ce cas, le procédé comporte aussi un lavage à l'eau, eau déionisée généralement comme pour la première variante. Toutefois ce lavage peut être moins poussé. On peut par exemple le réaliser jusqu'à l'obtention d'un filtrat présentant une conductivité d'au plus 2000 microsiemens cm$^{-1}$.

A l'issue de ce premier lavage à l'eau, on procède selon cette seconde variante à un second lavage ou un traitement du gâteau avec une solution acide ou de l'eau acidulée. Ce second lavage ou traitement a pour but d'obtenir à la fin de la préparation une silice ayant un pH d'au plus 8 et plus particulièrement compris entre 6,0 et 7,5 et aussi un PZC d'au moins 3 et plus particulièrement compris entre 4 et 6.

Le lavage ou traitement peut se faire par passage de la solution acide sur le gâteau, ou introduction de celle-ci dans la suspension obtenue après délitage du gâteau.

Ce lavage ou traitement acide est réalisé dans des conditions telles que pour obtenir une silice ayant le pH indiqué dans le paragraphe précédent, le pH de la suspension ou du milieu avant séchage doit se situer entre 4 et 8, notamment 5 et 8 et plus particulièrement 6 et 7.

Cette solution acide peut être par exemple une solution d'un acide minéral tel que l'acide nitrique.

Toutefois selon un mode de réalisation particulier, cette solution acide peut aussi être une solution d'un acide organique notamment d'un acide organique complexant. Cet acide pourra être choisi dans le groupe des acides carboxyliques, dicarboxyliques, hydroxycarboxyliques et amino-carboxyliques.

On peut citer comme exemple de tels acides l'acide acétique et pour les acides complexants, l'acide tartrique, l'acide maléique, l'acide glycérique, l'acide gluconique, l'acide citrique.

Il peut être avantageux, surtout dans le cas ne l'emploi d'une solution d'un acide minéral, de procéder à un ultime lavage par de l'eau déionisée.

A l'issue des lavages ou traitements selon cette seconde variante, on procède à un séchage de la même manière que décrit plus haut pour la première variante.

Selon une autre variante particulière, après la réaction acide-silicate et juste avant la séparation de la silice, on réalise un mûrissement de la suspension ou du gel. Ce mûrissement se fait généralement à un pH d'au plus 6 et compris entre 4 et 6 par exemple.

Il est aussi possible de réaliser un mûrissement en cours de réaction, par exemple à un pH entre 6 et 8. Ces mûrissements se font de préférence à chaud par exemple à une température comprise entre 80 et 100°C, et sur une durée qui peut varier entre quinze minutes et deux heures.

Enfin, on s'est aperçu qu'il était possible d'améliorer encore la compatibilité des produits de l'invention par un autre traitement complémentaire.

Le traitement consiste à utiliser un alcalino-terreux. Cet élément peut être introduit soit dans la suspension ou le gel de silice soit, de préférence sur le gâteau en particulier après délitage de celui-ci, sous forme de sel ou d'hydroxyde par exemple.

On utilise plus particulièrement un sel organique notamment complexant d'alcalino-terreux en général un sel de baryum, par exemple un acétate de baryum.

La quantité de silice selon l'invention utilisée dans les compositions dentifrices peut varier dans de larges limites, elle est habituellement comprise entre 5 et 35 %.

Les silices de l'invention s'appliquent particulièrement bien aux compositions dentifrices contenant au moins un élément choisi dans le groupe comprenant les fluorures, les phosphates, les guanidines dont la chlorhexidine. Elles peuvent en effet présenter une compatibilité selon les tests ci-après d'au moins 90% pour chacun de ces éléments.

Les silices de l'invention sont en outre compatibles avec les copolymères acide maléique-vinyléthyléther et peuvent donc aussi être incorporées à des compositions dentifrices comprenant ces copolymères. Elles peuvent enfin présenter une compatibilité avec le zinc d'au moins 50 % de préférence d'au moins 80 %.

En ce qui concerne les composés fluorés, leur quantité correspond de préférence à une concentration en fluor dans la composition comprise entre 0.01 et 1 % en poids et plus particulièrement 0,1 % à 0,5 %. Les composés fluorés sont en particulier les sels de l'acide monofluorophosphorique et particulièrement ceux de sodium, potassium, lithium, calcium, aluminium et ammonium, le mono et le difluorophosphate ainsi que des fluorures variés contenant le fluor sous forme d'ion lié particulièrement les fluorures alcalins comme ceux de sodium, lithium, potassium, le fluorure d'ammonium, le fluorure stanneux, le fluorure de manganèse, le fluorure de zirconium, le fluorure d'aluminium ainsi que des produits d'addition de ces fluorures entre eux ou avec d'autres fluorures, tels que les fluorures de potassium ou de sodium ou de manganèse.

D'autres fluorures sont également utilisables comme, par exemple, le fluorure de zinc, le fluorure de germanium, le fluorure de palladium, le fluorure de titane, les fluozirconates alcalins par exemple de sodium ou de potassium le fluozirconate stanneux, le fluoborate ou les fluosulfates de sodium, de potassium.

Les composés fluorés organiques peuvent également être utilisés, de préférence ceux connus comme les produits d'addition d'amines ou d'amino-acides à longue chaîne avec le fluorure d'hydrogène, le fluorure de cétylamine, le dihydrofluorure de bis-(hydroxyéthyl)aminopropyl N-hydroxyéthyl octadécylamine, le fluorure d'octadécylamine et le dihydrofluorure de N,N', N'tri-(polyoxyéthylène) N-hexadécylproplénodiamine.

En ce qui concerne le zinc, celui-ci est présent notamment sous forme citrate ou sulfate.

Pour les éléments utilisables comme agents anti-plaques du type polyphosphates ou polyphosphonates, guanidines, bis-biguanides on peut mentionner ceux indiqués dans les brevets US 3.934.002 ou 4.110.083 dont l'enseignement est incorporé ici.

Les compositions dentifrices peuvent contenir en outre un liant.

Les principaux liants utilisés sont notamment choisis parmi :
- les dérivés cellulosiques : méthylcellulose, hydroxyéthyl-cellulose, carboxyméthylcellulose sodique,
- les mucilages : carraghénates, alginates, agar-agar et géloses,
- les gommes : gommes arabique et adragante, gomme xanthane, gomme Karaya,
- les polymères carboxyvinyliques et acryliques,
- les résines de polyoxyéthylène.

Outre les silices de l'invention, les compositions dentifrices peuvent contenir aussi un ou plusieurs autres agents abrasifs polissants choisis notamment parmi :
- le carbonate de calcium précipité,
- le carbonate de magnésium,
- les phosphates de calcium, di-et tricalciques,
- le métaphosphate de sodium insoluble,
- le pyrophosphate de calcium,
- l'oxyde de titane (agent de blanchiment),
- les silicates,
- les alumines et silico-aluminates,
- les oxydes de zinc et d'étain,
- le talc,
- le kaolin.

Les compositions dentifrices peuvent aussi comprendre des détergents, des humectants, des agents aromatisants, édulcorants et des colorants et des conservateurs.

Les principaux détergents utilisés sont notamment choisis parmi :
- le laurylsulfate de sodium,
- le lauryléthersulfate et le laurylsulfoacétate de sodium,
- le dioctylsulfosuccinate de sodium,
- le laurylsarcosinate de sodium,
- le ricinoléate de sodium,
- les monoglycérides sulfatés.

Les principaux agents humectants utilisés sont notamment choisis parmi les polyalcools comme :
- le glycérol,
- le sorbitol, généralement en solution à 70 % dans l'eau,

- le propylène glycol.

Les principaux agents aromatisants (parfum) sont notamment choisis parmi : les essences d'anis, de badiane, de menthe, de baie de genièvre, de cannelle, de girofle et de rose.

Les principaux agents édulcorants sont notamment choisis parmi les imides orthosulfobenzoïques et les cyclamates.

Les principaux colorants utilisés sont notamment choisis selon la couleur désirée parmi :
- coloration rouge et rose : amaranthe, azorubine, cachou, coccine nouvelle (PONCEAU 4 R), cochenille, érythrosine,
- coloration verte : chlorophylle et chlorophylline,
- coloration jaune : jaune soleil (Orange S) et jaune de quinoléine.

Les principaux conservateurs les plus utilisés sont : les parahydroxybenzoates, le formol et les produits qui en dégagent, l'héxétidine, les ammoniums quaternaires, l'hexachlorophène, le bromophène et l'hexamédine.

Enfin, les compositions dentifrices contiennent des agents thérapeutiques dont les principaux sont notamment choisis parmi :
- les antiseptiques et les antibiotiques,
- les enzymes,
- les oligo-éléments et les composés fluorés qui ont été décrits ci-dessus.

Des exemples concrets mais non limitatifs de l'invention vont maintenant être donnés.

Auparavant les tests pour la mesure de la compatibilité de la silice avec différents éléments vont être décrits.

### Mesure de la compatibilité avec la chlorhexidine

4 g de silice sont dispersés dans 16 g d'une solution aqueuse de chlorhexidine de concentration 1 % en digluconate de chlorhexidine.

La suspension est agitée pensant 24 heures à 37°C.

La suspension est ensuite centrifugée à 20000 t/mn pendant 30 mn et le surnageant obtenu est filtré sur filtre Millipore 0,2 $\mu$m.

Par la suite 0,5 ml de solution ainsi filtrée est prélevé et dilué dans 100 ml d'eau dans une fiole jaugée. Cette solution constitue la solution de l'essai.

Une solution de référence est constituée selon le même protocole mais en absence de silice Une solution aqueuse à 1 % de digluconate de chlorhexidine est agitée pendant 24 heures à 37°C, puis est centrifugée à 20000 t/mn et le surnageant est filtré sur filtre Millipore de 0,2 $\mu$m. 0,5 ml de solution ainsi obtenue est dilué dans 100 ml d'eau dans une fiole jaugée.

On mesure ensuite l'absorbance des deux solutions à 254 nm à l'aide d'un spectrophotomètre (Uvikon 810/820).

La quantité de chlorhexidine libre notée % Compatibilité, est déterminée par le rapport :

$$\% \text{ Compatibilité} = \frac{\text{Absorbance de l'essai}}{\text{Absorbance de la référence}} \times 100$$

### Mesure de la compatibilité avec les fluorures

4 g de silice sont dispersés dans 16 g de solution à 0,3 % de fluorure de sodium (NaF). La suspension est agitée pendant 24 heures à 37°C. Après centrifugation de la suspension à 20000 t/mn pendant 30 mn, le surnageant est filtré sur filtre Millipore 0,2 $\mu$m. La solution ainsi obtenue, constitue la solution de l'essai.

Une solution de référence est constituée en utilisant le même protocole mais en absence de silice.

La compatibilité avec les fluorures est déterminée par le % de fluorure libre mesuré par électrode sélective à fluorure (Orion). Elle est déterminée par la relation ci-dessous.

$$\% \text{ Compatibilité} = \frac{\text{Concentration en F de l'essai (ppm)}}{\text{Concentration en F de la référence (ppm)}} \times 100$$

Mesure de la compatibilité avec le zinc

4 g de silice sont dispersés dans 100 ml de solution à 0,06 % de ZnSO$_4$, 7H$_2$0. On obtient une suspension dont le pH est stabilisé à 7 pendant 15 minutes par ajout de NaOH ou H$_2$SO$_4$ La suspension est agitée ensuite pendant 24 heures à 37°C puis est centrifugée à 20000 t/mn pendant 30 mn.

Le surnageant filtré sur filtre Millipore 0,2 $\mu$m, constitue la solution de l'essai.

Une solution de référence est constituée en suivant le même protocole mais en absence de silice.

La concentration en zinc libre des deux solutions est déterminée par absorption atomique (214 nm).

La compatibilité est déterminée par la relation ci-dessous :

$$\% \text{ Compatibilité} = \frac{\text{Concentration en Zn de l'essai (ppm)}}{\text{Concentration en Zn de la référence (ppm)}} \times 100$$

Mesure de la compatibilité avec les pyrophosphates de sodium et de potassium

4 g de silice sont dispersés dans 16 g de suspension à 1,5 % de pyrophosphate de sodium ou de potassium. La suspension est agitée pendant 24 heures à 37°C puis est centrifugée à 20000 t/mn pendant 30 mn.

Le surnageant est filtré sur filtre Millipore 0.2 $\mu$m. 0,2 g de solution diluée dans 100 ml d'eau dans une fiole jaugée, constitue la solution de l'essai.

Une solution de référence est constituée en suivant le même protocole mais en absence de silice.

La concentration en ion pyrophosphate (P$_2$O$_7$$^{-}$ $^{-}$) libre des deux solutions est déterminée par chromatographie ionique (système DIONEX 2000) équipé d'un intégrateur.

La compatibilité est déterminée par le rapport des aires des pics obtenus sur les chromatogrammes et correspondant au temps de rétention du pyrophosphate, de l'essai et de la référence.

$$\% \text{ Comptabilité} = 100 \times \frac{\text{aire du pic de l'essai}}{\text{aire du pic de la référence}}$$

EXEMPLE 1

Cet exemple concerne la préparation d'une silice compatible du type abrasive.

Dans un réacteur équipé d'un système de régulation de température et de pH et d'un système d'agitation par turbine, on introduit 6 l d'eau déionisée.

Après la mise en marche de l'agitation (300 t/mn), le pied de cuve ainsi constitué est chauffé à 85°C.

Lorsque la température est atteinte, on procède à l'addition simultanée de 8,5 l de silicate de sodium de concentration en silice de 120 g/l, de rapport SiO$_2$/NaO$_2$ égal à 3,5 et de débit 0,34 1/mn et de 13,5 l d'acide sulfurique de concentration 80 g/l. Le débit d'acide est ajusté de manière à maintenir le pH du milieu à une valeur constante de 8,0.

Après 40 mn d'addition, on arrête l'addition de silicate et on continue l'addition d'acide jusqu'à stabiliser le pH du mélange réactionnel à 4.

On réalise par la suite, un mûrissement de 15 mn à ce pH et à 85°C.

Le mélange est ensuite filtré et le gâteau humide est lavé à l'eau déionisée jusqu'à ce que la conductivité du filtrat soit inférieure à 100 microsiemens.

On procède ensuite à un lavage du gâteau avec de l'eau amenée à pH 4 par ajout d'acide acétique.

Un dernier lavage est réalisé avec de l'eau déionisée.

Le produit est ensuite séché par atomisation et broyé sur un broyeur de type forplex pour obtenir une granulométrie de 10 microns.

Les caractéristiques physico-chimiques de la silice ainsi obtenue sont les suivantes :

| Surface BET | 100 m2/g |
|---|---|
| Surface CTAB | 55 m2/g |
| Prise d'huile | 120 cm3/100g |
| pH | 6,5 |

Les analyses chimiques de la silice sont regroupées dans le tableau ci-dessous.

| ions | SO$_4$ | Al | Fe | Ca | C |
|---|---|---|---|---|---|
| ppM | 100 | 250 | 130 | 300 | 10 |

La chimie de surface est quantifiée par les paramètres suivants :
Ho supérieur à 3,3
PZC = 4
$\Delta_\gamma$ = 5 cm$^{-1}$
nombre de OH/nm2 : 9.
Dans le tableau ci-dessous sont données les compatibilités de la silice avec les différents ingrédients d'une formulation dentifrice.

| Ingrédients | Fluorure NaF | Pyrophosphate Na/K | Chlorhexidine digluconate | Zinc ZnSO$_4$ |
|---|---|---|---|---|
| % Compatibilité | 95 | 98 | 75 | 70 |

EXEMPLE 2

On reprend l'exemple 1 pour obtenir un gâteau de silice lavé à une eau de pH 4 par ajout d'acide acétique.
Le gâteau ainsi obtenu, est délité dans un déliteur pour obtenir une suspension fluide.
On ajoute 0,2 g d'acétate de baryum sous agitation.
La silice est ensuite séchée par atomisation puis broyée pour obtenir une taille moyenne de particule de 10 $\mu$m.
Les caractéristiques physico-chiomiques de la silice ainsi obtenue sont les suivantes :

| Surface BET | 100 m2/g |
|---|---|
| Surface CTAB | 60 m2/g |
| Prise d'huile | 110 cm3/100g |
| pH | 6,5 |

Les analyses chimiques de la silice sont regroupées dans le tableau ci-dessous.

| ions | SO$_4$ | Al | Fe | Ca | C |
|---|---|---|---|---|---|
| ppM | 100 | 250 | 130 | 400 | 40 |

La chimie de surface est quantifiée par les paramètres suivants :
Ho supérieur a 3,3
PZC = 4,5
$\Delta_\gamma$ = 2 cm$^{-1}$
nombre de OH/nm2 : 8

| Compatibilités | | | | |
|---|---|---|---|---|
| ingrédients | Fluorure NaF | Pyrophosphate Na/K | Chlorhexidine digluconate | Zinc Zn SO$_4$ |
| % compatible | 95 | 98 | 90 | 70 |

EXEMPLE 3

Cet exemple concerne la préparation d'un gel de silice épaississante.

Dans un réacteur équipé d'un système de régulation de température et de pH et d'un système d'agitation par turbine, on introduit 14 l de silicate de Na de concentration en silice de 86 g/l et de rapport SiO$_2$/Na$_2$O de 3,5.

Après la mise en marche de l'agitation (100 t/mn), on procède à l'addition de 1,45 l d'ammoniaque à 28 % en 2 mn.

On ajoute par la suite 0,8 l d'acide sulfurique de concentration 200 g/l avec un débit de 0,2 l/min.

A ce niveau, on laisse réagir le mélange pendant 5 mn à température contrôlée de 20°C.

On ajoute ensuite 5,2 l d'acide sulfurique de concentration 200 g/l avec un débit de 0,2 l/mn.

Après apparition du gel (visuel ou par mesure de turbitité), on laisse le mélange mûrir pendant 10 mn.

Le gel est dispersé par la suite en agitant le mélange à 400 t/mn pendant 30 mn.

Le pH du milieu est ensuite descendu à la valeur 3,5 par addition d'acide sulfurique (200 g/l) avec un débit de 0,2 l/mn.

On laisse se stabiliser le mélange réactionnel pendant 1heure à une température de 60°C.

On achève la préparation du gel en filtrant ce mélange et en procédant au lavage du gâteau obtenu par 2 fois 20 l d'eau déionisée de température 60°C et par 20 l d'eau de pH 3.

Le traitement selon l'invention est réalisé ensuite par un lavage effectué avec de l'eau déionisée et à 20°C jusqu'à obtenir une conductivité de 200 microsiemens cm$^{-1}$.

Le gâteau obtenu est délité pour former une suspension homogène de silice de concentration 10 % ajustée par ajout d'eau.

La silice est séchée par atomisation sur un atomiseur de type Anhydro. La silice est ensuite micronisée sur un broyeur JET Pulverizer pour avoir une granulométrie de 1,5 $\mu$m.

Les caractéristiques physico-chimiques de la silice ainsi obtenue sont les suivantes :

| | |
|---|---|
| Surface BET | 450 m2/g |
| Surface CTAB | 350 m2/g |
| Prise d'huile | 300 cm3/100g |
| pH | 6,8 |
| Densité apparente | 0,270 |
| Indice de réfraction | 1,445 |

Les analyses chimiques de la silice sont regroupées dans le tableau ci-dessous.

| ions | SO$_4$ | Al | Fe | Ca | C |
|---|---|---|---|---|---|
| ppM | 500 | 200 | 120 | 300 | 10 |

La chimie de surface est quantifiée par les paramètres suivantes :
Ho supérieur à 3,3
PZC = 3,6
Pas de bandes d'adsorption de la pyridine.

| Compatibilités | | | | |
|---|---|---|---|---|
| ingrédients | Fluorure NaF | Pyrophosphate NaK | Chlorhexidine digluconate | Zinc Zn SO$_4$ |
| % compatible | 90 | 95 | 65 | 50 |

EXEMPLE 4

Cet exemple concerne une silice épaississante.

Dans un réacteur équipé d'un système de régulation de pH et de température, on introduit 6 l d'eau puis on ajoute sous agitation 150 g de sulfate de sodium.

Le mélange est chauffé à 60°C. On procède à l'addition simultanée de 10 l de silicate de sodium (Rm = 3,5 et SiO$_2$ = 220 g/l) et d'acide sulfurique de concentration 80 g/l en 40 mn.

Le débit d'acide sulfurique est ajusté de manière à maintenir le pH du milieu réactionnel à une valeur constante de 7,8.

Le pH du milieu est stabilisé à la valeur 4,0 par ajout très rapide d'acide sulfurique. On laisse mûrir pendant 15 mn à 60°C.

On filtre le mélange réactionnel à 60°C et on procède au lavage du gâteau de silice par de l'eau déionisée de manière à avoir une conductibilité du filtrat de 900 microsiemens.cm$^{-1}$. On lave ensuite le gâteau avec de l'eau de pH = 4 ajusté par addition d'acide acétique et on procède à un dernier lavage à l'eau déionisée.

Le gâteau de silice ainsi obtenu est délité puis on y ajoute sous agitation 2 g d'acétate de calcium.

On sèche la silice par atomisation et on micronise sur un Jet Pulverizer pour ajuster la taille des particules de silice à 1,5 $\mu$m.

Les caractéristiques physico-chimiques de la silice ainsi obtenues sont les suivantes :

| | |
|---|---|
| Surface BET | 320 m2/g |
| Surface CTAB | 120 m2/g |
| Prise d'huile | 250 cm3/100g |
| pH | 6,5 |

Les analyses chimiques de la silice sont regroupées dans le tableau ci-dessous.

| ions | SO$_4$ | Al | Fe | Ca | C |
|---|---|---|---|---|---|
| ppM | 100 | 200 | 120 | 300 | 20 |

La chimie de surface est quantifiée par les paramètres suivants :
Ho supérieur à 3,3
PZC = 4
nombre de OH/nm2 = 11
Pas de bandes d'adsorption de la pyridine

| Compatibilités | | | | |
|---|---|---|---|---|
| ingrédients | Fluorure NaF | Pyrophosphate Na/K | Chlorhexidine digluconate | Zinc ZnSO$_4$ |
| % compatible | 90 | 95 | 90 | 80 |

EXEMPLE 5

Cet exemple concerne la préparation d'une silice abrasive.

Dans un réacteur équipé d'un système de régulation de température et de pH et d'un système d'agitation par turbine, on introduit 6 l d'eau déionisée.

Après la mise en marche de l'agitation (300 t/mn), le pied de cuve ainsi constitué est chauffé à 85°C.

Lorsque la température est atteinte, on procède à l'addition simultanée de 8,5 l de silicate de sodium de concentration en silice de 120 g/l, de rapport $SiO_2/Na_2O$ égal à 3,5 et de débit 0,34 l/mn et de 13,5 l d'acide sulfurique de concentration 80 g/l. Le débit d'acide est ajusté de manière à maintenir le pH du milieu à une valeur constante de 8,0.

Après 40 mn d'addition, on arrête l'addition de silicate et on laisse mûrir le mélange pendant 15 mn à 85°C et pH 8.

On continue l'addition d'acide jusqu'à stabiliser le pH du mélange réactionnel à 4.

On réalise par la suite, un mûrissement de 15 mn à ce pH et à 85°C.

Le mélange est ensuite filtré et le gâteau humide est lavé à l'eau déionisée jusqu'à ce que la conductivité du filtrat soit de 100 microsiemens.cm$^{-1}$.

On procède ensuite à deux lavages du gâteau avec de l'eau amenée à pH 4 par ajout d'acide acétique.

Un dernier lavage est réalisé avec de l'eau déionisée.

Le produit est ensuite séché par atomisation et broyé sur un broyeur de type forplex pour obtenir une granulométrie de 9,0 microns.

Les caractéristiques physico-chimiques de la silice ainsi obtenue sont les suivantes :

| | |
|---|---|
| - Surface BET | 60 m2/g |
| - Surface CTAB | 50 m2/g |
| - Prise d'huile | 120 cm3/100g |
| - pH | 6,0 |

Les analyses chimiques de la silice sont données ci-dessous :

| Ions | SO₄ | Al | Fe | Ca | C |
|---|---|---|---|---|---|
| ppM | 100 | 250 | 100 | 200 | 10 |

La chimie de surface est quantifiée par les paramères suivants :
Ho supérieur à 3,3
PZC = 4,5
pas de bandes d'adsorption de la pyridine.

| Compatibilité | | | | |
|---|---|---|---|---|
| Ingrédients | Fluorure NaF | Pyrophosphate Na/K | Chlorhexidine digluconate | Zinc ZnSO₄ |
| % compatible | 95 | 98 | 70 | 80 |

EXEMPLE 6

Cet exemple concerne la préparation d'une silice du type épaississante dans un réacteur équipé d'un système d'agitation par turbine, on introduit 5,07 l de silicate de sodium de concentration en silice de 120 g/l et de rapport $SiO_2/Na_2O$ = 3,5 et 3,8 l d'eau déionisée.

Après la mise en marche de l'agitation (300 tr/mn), le pied de cuve ainsi constitué est chauffé à 68°C.

Lorsque la température est atteinte, on procède à l'addition de 2,64 l d'acide sulfurique de concentration 80,5 g/l. Le débit d'acide est de 0,120 l/mn.

Après 22 mn d'addition, on arrête l'addition d'acide et on laisse mûrir le mélange pendant 10 mn (une augmentation brusqeu de la turbitité est observée).

On procède par la suite, à une addition de 4,2 l d'acide sulfurique en 35 mn.

La température est ensuite portée à 87°C et on réalise une addition simultanée de silicate de sodium avec un débit de 30 ml/mn et d'acide sulfurique avec un débit de 52 ml/mn pendant 30 mn.

La température est portée à 95°C et on ajoute 0,523 l d'acice sulfurique en 10 mn.

On laisse, par la suite mûrir le mélange pendant 10 mn.

Enfin, on amène le pH du milieu à 4 par ajout d'acide.

Le mélange est ensuite filtré et le gâteau humide est lavé à l'eau déionisée jusqu'à ce que la conductivité du filtrat soit de 100 microsiemens cm$^{-1}$.

On procède ensuite à un lavage du gâteau avec de l'eau amenée à pH 4 par ajout d'acide acétique.

Un dernier lavage est réalisé avec de l'eau déionisée.

Le produit est ensuite séché par atomisation et micronisé sur un broyeur de type Jet Pulverizer pour obtenir une granulométrie de 1,2 microns.

Les caractéristiques physico-chimiques de la silice ainsi obtenue sont les suivantes :

| | |
|---|---|
| Surface BET | 180 m2/g |
| Surface CTAB | 170 m2/g |
| Prise d'huile | 350 cm3/100g |
| pH | 6,8 |

Les analyses chimiques de la silice sont regroupées dans le tableau ci-dessous.

| ions | SO$_4$ | Al | Fe | Ca | C |
|---|---|---|---|---|---|
| ppM | 500 | 200 | 120 | 300 | 10 |

La chimie de surface est quantifiée par les paramètres suivants :

Ho supérieur à 3,3

PZC = 3,6

Pas de bandes d'adsorption de la pyridine.

| Compatibilité | | | | |
|---|---|---|---|---|
| Ingrédients | Fluorure NaF | Pyrophosphate Na/K | Chlorhexidine digluconate | Zinc ZnSO$_4$ |
| % compatible | 90 | 95 | 70 | 60 |

EXEMPLE 7

On décrit dans cet exemple la préparation d'un gâteau de silice qui servira de produit de départ pour les silices des exemples 8 à 12.

Dans un réacteur de 30 l équipé d'un système de régulation de température et de pH et d'un système d'agitation type Mixel, on introduit 1,4 l de silicate de sodium de rapport SiO$_2$/Na$_2$O 3,45 et de concentration en SiO$_2$ de 135 g/l, préchauffé à 75°C. Après la mise en marche de l'agitation (300 t/mn), le pied de cuve ainsi constitué est chauffé à 85°C.

Lorsque la température est atteinte, on procède à l'addition simultanée de ce même silicate de sodium avec un débit de 0,28 l/mn et d'acide sulfurique de concentration 80 g/l préchauffé à 75°C avec un débit de 0,16 l/mn.

Le pH moyen du milieu au cours de l'addition simultanée est de 9,8.

Après 47 mn d'addition simultanée, on arrête l'addition de silicate et on continue l'addition d'acide avec ce même débit jusqu'à atteindre un pH de 8. A ce niveau, on porte la température du mélange réactionnel à 95°C en 10 mn tout en continuant l'addition d'acide pour stabiliser le pH du mélange réactionnel à 4,2 dans ce même temps.

On réalise par la suite, un mûrissement de 15 mn à ce pH et à 95°C.

Le mélange est ensuite filtré et le gâteau humide est lavé à l'eau déionisée jusqu'à ce que la conductivité du filtrat soit de 2000 microsiemens cm$^{-1}$.

Le gâteau obtenu à ce niveau servira de base pour élaborer les silices à chimie de surfaces contrôlée des exemples 8 à 12.

EXEMPLE 8

On reprend le gâteau obtenu à l'exemple 7.

Ce gâteau est dispersé dans de l'eau déionisée pour former une suspension à 100 g/l de silice et la suspension est filtrée par la suite. On renouvelle l'opération jusqu'à obtenir une conductivité du filtrat de 100 microsiemens cm$^{-1}$.

Le gâteau est ensuite redispersé sous forme de suspension à 150 g/l dans de l'eau déionisée et le pH de cette dernière est amené à 6 par ajout d'acide acetique.

Après filtration, un dernier lavage est réalisé avec de l'eau déionisée.

Le produit est ensuite séché par atomisation et broyé sur un broyeur de type forplex pour obtenir une granulométrie de 9,0 $\mu$m.

EXEMPLE 9

Un gâteau obtenu selon le mode opératoire de l'exemple 7 est lavé à l'eau déionisée jusqu'à obtenir une conductibilité du filtrat de 500 microsiemens.cm$^{-1}$.

On lave ensuite le gâteau avec 10 l d'eau amenée à pH 4 par ajout d'acide gluconique.

On procède ensuite à un dernier lavage avec de l'eau déionisée.

Le gâteau est délité pour obtenir une suspension homogène et on ajoute sous agitation 8,5 g d'acétate de baryum (Ba (C2H2O2, H2O). Le mélange est laissé mûrir pendant 30 mn.

Le produit est ensuite séché par atomisation et broyé sur un broyeur de type forplex pour obtenir une granulométrie de 9,0 $\mu$m.

EXEMPLE 10

Un gâteau obtenu selon le mode opératoire de l'exemple 7 est lavé à l'eau déionisée jusqu'à obtenir une conductibilité du filtrat de 500 microsiemens.cm$^{-1}$.

Le gâteau est ensuite redispersé sous forme de suspension à 150 g/l dans de l'eau déionisée et le pH de cette dernière est amené à 6 par ajout d'acide acétique.

On ajoute sous agitation, 25 g d'hydroxyde de barium Ba(OH)2, 8H2O) et le mélange est laissé mûrir pendant 30 mn.

La suspension est ensuite filtrée et lavée avec 5 l d'eau.

Le produit est ensuite séché par atomisation et broyé sur un broyeur de type forplex pour obtenir une granulométrie de 9,0 microns.

EXEMPLE 11

Un gâteau obtenu selon le mode opératoire de l'exemple 7 est lavé à l'eau déionisé jusqu'à obtenir une conductibilité du filtrat de 100 microsiemens.cm$^{-1}$.

Le gâteau est ensuite redispersé sous forme de suspension à 150 g/l dans de l'eau déionisée et le pH de cette dernière est amené à 6,3 par ajout d'acide acétique.

On procède ensuite à un dernier lavage avec de l'eau déionisée.

On ajoute sous agitation, 0,1 g d'acétate de baryum Ba(C2H2O2)2, H2O) et le mélange est laissé mûrir pendant 30 mn.

Le produit est ensuite séché par atomisation et broyé sur un broyeur de type forplex pour obtenir une granulométrie de 9,0 microns.

EXEMPLE 12

On reprend le gâteau de l'exemple 7. Ce gâteau est dispersé dans de l'eau déionisée pour former une suspension à 100 g/l de silice et la suspension est filtrée par la suite. On renouvelle l'opération jusqu'à obtenir une conductivité du filtrat de 200 microsiemens.cm$^{-1}$.

Le produit est ensuite séché par atomisation et broyé sur un broyeur de type forplex pour obtenir une granulométrie de 9,0 microns.

Les caractéristiques des silices des exemples 8 à 12 sont données dans les tableaux ci-dessous :

16

| Exemple | Surfaces | | pH | OH/nm2 | Prise d'huile | PZC | Ho Indice de réfraction |
|---|---|---|---|---|---|---|---|
| | BET | CTAB | | | | | |
| | m2/g | | | | | | |
| 8 | 250 | 50 | 6,9 | 12 | 102 | 4,5 >3,3 | 1,460 |
| 9 | 250 | 45 | 6,8 | 10 | 110 | 4 >3,3 | 1,457 |
| 10 | 250 | 60 | 7,2 | 12 | 105 | 3,8 >3,3 | 1,458 |
| 11 | 260 | 55 | 7,0 | 10 | 105 | 6 >3,3 | 1,457 |
| 12 | 250 | 45 | 7,5 | 12 | 100 | 3,6 >3,3 | 1,446 |

| Analyse chimique | | | | | |
|---|---|---|---|---|---|
| | Ions (ppM) | | | | |
| Exemples | SO4 | Al | Fe | Ca | C |
| 8 | 70 | 300 | 150 | 300 | 20 |
| 9 | 150 | 350 | 200 | 350 | 20 |
| 10 | 200 | 400 | 200 | 350 | 50 |
| 11 | 50 | 230 | 120 | 120 | 20 |
| 12 | 200 | 415 | 200 | 355 | 20 |

| Compatibilités | | | | |
|---|---|---|---|---|
| Exemples | Fluorure NaF | Pyrophosphate Na/K | Chlorhexidine digluconate | Zinc $ZnSO_4$ |
| 8 | 90 | 95 | 75 | 70 |
| 9 | 95 | 98 | 80 | 85 |
| 10 | 96 | 95 | 70 | 65 |
| 11 | 95 | 96 | 98 | 80 |
| 12 | 95 | 95 | 70 | 60 |

EXEMPLE 13 COMPARATIF

A titre comparatif on donne dans le tableau ci-dessous les mesures de compatibilité de silices commerciales généralement utilisées dans les formations dentifrices, ainsi que leurs caractéristiques physico-chimiques.

Les mesures sont effectuées selon les tests décrits par ailleurs.

| Silices Marque Fabriquant | Surface | m2/g | Ho | OH/nm2 | SO$_4$ % en poids | Compatibilité | | | |
|---|---|---|---|---|---|---|---|---|---|
| | CTAB | BET | | | | CHx | F | Zn | PYR |
| Zeodent 113 Hubert | 50 | 100 | <3 | 30 | 0,65 | 1 | 95 | 0 | 95 |
| Z113 Zeofinn | 70 | 175 | <3 | 17 | 0,50 | 1 | 96 | 0 | 95 |
| Syloblanc 81 Grace | 240 | 400 | <3 | 17 | 1,56 | 0 | 90 | 40 | 95 |
| Syloid 244 Grace | | 400 | <3 | 17 | 0,7 | 4 | 90 | 2 | 90 |
| Sipernat 22S Degussa | 180 | 190 | <3 | 16 | 1,0 | 0 | 90 | 20 | 90 |
| Tixosil 53 Rhône Poulenc | 50 | 250 | <3 | 30 | 0,8 | 0 | 60 | 0 | 0 |
| CHx = chlorhexidine, F = fluor, Zn = zinc, Pyr = pyrophosphate. | | | | | | | | | |

On notera que pour tous les produits mentionnés dans le tableau le PZC est inférieur à 3.

EXEMPLE 14

Cet exemple concerne la formulation d'une pâte dentifrice translucide du type gel avec des silices de l'invention.

La formule est la suivante :

| | |
|---|---|
| Sorbitol (70 % aqueux) | 65,00 |
| Glycérine | 0,00 |
| CMC 7mFD | 0,80 |
| Saccharinate de sodium | 0,20 |
| Fluorure de sodium | 0,24 |
| Benzoate de sodium | 0,08 |
| Arôme | 2,00 |
| Silice abrasive exemple 2 | 15,00 |
| Silice épaississante exemple 6 | 8,00 |
| Digluconate de chlorhexidine | 1,00 |
| Eau distillée | 7,68 |

Le dentifrice présente des propriétés rhéologiques satisfaisantes et s'extrude convenablement initialement et après stockage (2 mois).

L'expertise visuelle du dentifrice confirme que la tenue de l'extrudat est correct et qu'il se présente sous forme d'un gel translucide.

Ce dentifrice présente en outre les propriétés suivantes :

| | |
|---|---|
| pH dilution à 10 % | 6,8 |
| Pouvoir abrasif sur cuivre norme LNE (mg) | 5,6 |
| Viscosité plastique (Pa.s) | 0,5 |

Une activité anti bactérienne est constatée.

EXEMPLE 15

Cet exemple concerne la formulation d'un dentifrice opaque du type pâte :
La formule est la suivante :

| | |
|---|---|
| Glycérine | 22,OO |
| CMC 7mFD | 1,0 |
| Saccharinate de sodium | 0,20 |
| Monofluorophosphate de sodium | 0,76 |
| Fluorure de sodium | O,10 |
| Lauryl sulfate de sodium (30 % aqueux) | 4,67 |
| Benzoate de sodium | 0,10 |
| Arôme | 0,90 |
| Dioxyde de titane | 1,00 |
| Silice abrasive exemple 5 | 31,50 |
| $ZnSO_4$, 7 $H_2O$ | 0,48 |
| Eau distillée | 37,29 |

L'expertise rhéologique et visuelle de la pâte dentifrice obtenue montre que les propriétés usuelles du dentifrice sont bonnes.

**Revendications**

1. Silice caractérisée en ce qu'elle présente une compatibilité avec les produits du type guanidines et en particulier la chlorhexidine d'au moins 65 % et plus particulièrement d'au moins 90% et en ce qu'elle présente une chimie de surface telle que sa fonction d'acidité Ho soit supérieure à 3,3.

2. Silice selon la revendication 1 caractérisée en ce qu'elle présente en outre une compatibilité avec le zinc d'au moins 50 % de préférence 80 %.

3. Silice selon la revendication 1 caractérisée en ce qu'elle possède un spectre infrarouge d'adsorption de la pyridine présentant par rapport au spectre de la pyridine seule pour la bande de 1440 $cm^{-1}$ un déplacement d'au plus 10 $cm^{-1}$, plus particulièrement d'au plus 5 $cm^{-1}$.

4. Silice selon la revendication 3 caractérisée en ce que le déplacement précité est nul.

5. Silice selon l'une des revendications précédentes, caractérisée en ce qu'elle présente une teneur en anions du type $SO_4^{2-}$, $Cl^-$, $NO_3^-$ $PO_4^{3-}$, $CO_3^{2-}$ d'au plus $5.10^{-3}$ moles pour 100 g de silice.

6. Silice selon la revendication 5, caractérisé en ce qu'elle présente une teneur en anions précités d'au plus $1.10^{-3}$, plus particulièrement d'au plus $0,2.10^{-3}$ moles pour 100 g de silice.

7. Silice selon la revendication 5 ou 6 caractérisée en ce qu'elle présente une teneur en sulfate exprimé en $SO_4$ d'au plus 0,5%, de préférence d'au plus 0,1 % et plus particulièrement d'au plus 0,02 %.

8. Silice selon l'une des revendications précédentes caractérisé en ce qu'elle présente une chimie de surface telle que le nombre de OH exprimé en OH/nm2 soit d'au plus 15 et plus particulièrement d'au plus 12.

9. Silice selon l'une des revendications précédentes caractérisée en ce qu'elle présente un point de charge nulle (PZC) d'au moins 3, plus particulièrement compris entre 4 et 6.

10. Silice selon l'une des revendications précédentes, caractérisée en ce qu'elle présente une teneur en aluminium d'au plus 500 ppm.

11. Silice selon l'une des revendications précédentes, caractérisée en ce qu'elle présente une teneur en fer d'au plus 200 ppm.

**12.** Silices selon l'une des revendications précédentes caractérisée en ce qu'elle présente une teneur en calcium d'au plus 500 ppm, en particulier d'au plus 300 ppm.

**13.** Silice selon l'une des revendications précédentes, caractérisée en ce qu'elle présente une teneur en carbone d'au plus 50 ppm et plus particulièrement d'au plus 10 ppm.

**14.** Silice selon l'une des revendications précédentes, caractérisée en ce qu'elle présente un pH d'au plus 8 et plus particulièrement compris entre 6,0 et 7,5.

**15.** Silice selon l'une des revendications précedentes, caractérisée en ce qu'elle présente une surface BET comprise entre 40 et 600 m2/g.

**16.** Silice selon l'une des revendications précédentes, caractérisée en ce qu'elle présente une surface CTAB comprise entre 40 et 400 m2/g.

**17.** Silice selon l'une des revendications précédentes, du type abrasif caractérisée en ce qu'elle présente une surface BET comprise entre 40 et 300 m2/g.

**18.** Silice selon la revendication 17, caractérisée en ce qu'elle présente une surface CTAB comprise entre 40 et 100 m2/g.

**19.** Silice selon l'une quelconque des revendications 1 à 16, du type épaississant caractérisée en ce qu'elle présente une surface BET comprise entre 120 et 450 m2/g plus particulièrement 120 et 200 m2/g.

**20.** Silice selon la revendication 19, caractérisée en ce qu'elle présente une surface CTAB comprise entre 120 et 400 m2/g.

**21.** Silice selon l'une quelconque des revendications 1 à 16, du type bifonctionnelle, caractérisée en ce qu'elle présente une surface BET comprise entre 80 et 200 m2/g.

**22.** Silice selon la revendication 21, caractérisée en ce qu'elle présente une surface CTAB comprise entre 80 et 200 m2/g.

**23.** Silice selon l'une des revendications précédentes, caractérisée en ce qu'elle présente une prise d'huile comprise entre 80 et 500 cm3/100 g.

**24.** Silice selon l'une des revendications précédentes, caractérisée en ce qu'elle présente une taille moyenne de particules comprise entre 1 et 10 $\mu$m.

**25.** Silice selon l'une quelconque des revendications précédentes, caractérisée en ce qu'elle présente une densité apparente comprise entre 0,01 et 0,3.

**26.** Silice selon l'une des revendications précédentes caractérisée ence qu'il s'agit d'une silice de précipitation.

**Claims**

**1.** A silica, characterised in that it has a compatibility with products of the guanidine type, and, in particular, chlorhexidine, of at least 65%, and, more particularly, of at least 90%, and in that it has a surface chemistry such that its acidity function Ho is greater than 3.3.

**2.** A silica according to Claim 1, characterised in that it also has a compatibility with zinc of at least 50%, preferably of 80%.

**3.** A silica according to Claim 1, characterised in that it has an infra-red pyridine adsorption spectrum, which, compared with the pyridine spectrum alone for the band of 1440 cm$^{-1}$, has a displacement of at the most 10 cm$^{-1}$, more particularly of at the most 5 cm$^{-1}$.

4. A silica according to Claim 3, characterised in that the afore-said displacement is zero.

5. A silica according to one of the preceding claims, characterised in that it has an anion content of the type $SO_4{}^{2-}$, $Cl^-$, $NO_3{}^-PO_4{}^{3-}$, $CO_3{}^{2-}$ of at the most $5.10^{-3}$ moles for 100 g of silica.

6. A silica according to Claim 5, characterised in that it has a content of the afore-mentioned anions of at the most $1.10^{-3}$, more particularly of at the most $0.2.10^{-3}$ moles for 100 g of silica.

7. A silica according to Claim 5 or Claim 6, characterised in that it has a sulphate content, expressed in $SO_4$, of at the most 0.5%, preferably of at the most 0.1%, and more particularly of at the most 0.02%.

8. A silica according to one of the preceding claims, characterised in that it has a surface chemistry such that the number of OH, expressed in $OH/nm^2$, is at the most 15, and, more particularly, at the most 12.

9. A silica according to one of the preceding claims, characterised in that it has a point zero charge (PZC) of at least 3, more particularly of between 4 and 6.

10. A silica according to one of the preceding claims, characterised in that it has an aluminium content of at the most 500 ppm.

11. A silica according to one of the preceding claims, characterised in that it has an iron content of at the most 200 ppm.

12. A silica according to one of the preceding claims, characterised in that it has a calcium content of at the most 500 ppm, in particular of at the most 300 ppm.

13. A silica according to one of the preceding claims, characterised in that it has a carbon content of at the most 50 ppm, and more particularly of at the most 10 ppm.

14. A silica according to one of the preceding claims, characterised in that it has a pH of at the most 8, and more particularly of between 6.0 and 7.5.

15. A silica according to one of the preceding claims, characterised in that it has a BET surface of between 40 and 600 $m^2/g$.

16. A silica according to one of the preceding claims, characterised in that it has a CTAB surface of between 40 and 400 $m^2/g$.

17. A silica according to one of the preceding claims, of the abrasive type, characterised in that it has a BET surface of between 40 and 300 $m^2/g$.

18. A silica according to Claim 17, characterised in that it has a CTAB surface of between 40 and 100 $m^2/g$.

19. A silica according to any one of Claims 1 to 16, of the thickening type, characterised in that it has a BET surface of between 120 and 450 $m^2/g$, more particularly of between 120 and 200 $m^2/g$.

20. A silica according to Claim 19, characterised in that it has a CTAB surface of between 120 and 400 $m^2/g$.

21. A silica according to any one of Claims 1 to 16, of the bifunctional type, characterised in that it has a BET surface of between 80 and 200 $m^2/g$.

22. A silica according to Claim 21, characterised in that it has a CTAB surface of between 80 and 200 $m^2/g$.

23. A silica according to one of the preceding claims, characterised in that it has an oil sample of between 80 and 500 $cm^3/100$ g.

24. A silica according to one of the preceding claims, characterised in that it has an average particle size of between 1 and 10 $\mu$m.

25. A silica according to any one of the preceding claims, characterised in that it has an apparent density of between 0.01 and 0.3.

26. A silica according to one of the preceding claims, characterised in that it is a precipitation silica.

**Patentansprüche**

1. Kieselsäure, dadurch gekennzeichnet, daß sie mit Produkten vom Guanidintyp, und insbesondere mit Chlorhexidin, wenigstens zu 65 %, und insbesondere wenigstens zu 90 %, verträglich ist, und eine Oberflächenchemie mit einer Acidität Ho von über 3,3 besitzt.

2. Kieselsäure nach Anspruch 1, dadurch gekennzeichnet, daß sie ferner mit Zink zu wenigstens 50 %, vorzugsweise 80 %, verträglich ist.

3. Kieselsäure nach Anspruch 1, dadurch gekennzeichnet, daß sie ein IR-Absorptionspektrum des Pyridins aufweist, bei dem im Vergleich zum Spektrum des Pyridins allein die Bande bei 1.440 $cm^{-1}$ um höchstens 10 $cm^{-1}$, insbesondere um höchstens 5 $cm^{-1}$ versetzt ist.

4. Kieselsäure nach Anspruch 3, dadurch gekennzeichnet, daß die Verschiebung gleich 0 ist.

5. Kieselsäure nach einem der vorhergehenden Ansprüche, dadurch gekennzeichnet, daß sie einen Gehalt an Anionen vom Typ $SO_4{}^{2-}$, $Cl^-$, $NO_3{}^-$, $PO_4{}^{3-}$, $CO_3{}^{2-}$ von höchstens $5 \bullet 10^{-3}$ mol pro 100 g Kieselsäure aufweist.

6. Kieselsäure nach Anspruch 5, dadurch gekennzeichnet, daß der Gehalt der vorstehenden Anionen höchstens $1 \bullet 10^{-3}$, insbesondere höchstens $0,2 \bullet 10^{-3}$ mol pro 100 g Kieselsäure, beträgt.

7. Kieselsäure nach Anspruch 5 oder 6, dadurch gekennzeichnet, daß sie einen Sulfatgehalt, ausgedrückt als $SO_4{}^{2-}$ von höchstens 0,5 %, insbesondere von höchstens 0,1 % und am besten von höchstens 0,02 % enthält.

8. Kieselsäure nach einem der vorhergehenden Ansprüche, dadurch gekennzeichnet, daß sie eine Oberflächenchemie aufweist, bei der die Zahl der OH-Gruppen, ausgedrückt als $OH/nm^2$ höchstens 15, insbesondere höchstens 12, beträgt.

9. Kieselsäure nach einem der vorhergehenden Ansprüche, dadurch gekennzeichnet, daß sie einen isoelektrischen Punkt (PZC) von wenigstens 3, insbesondere zwischen 4 und 6, besitzt.

10. Kieselsäure nach einem der vorhergehenden Ansprüche, dadurch gekennzeichnet, daß sie einen Aluminiumgehalt von höchstens 500 ppm besitzt.

11. Kieselsäure nach einem der vorhergehenden Ansprüche, dadurch gekennzeichnet, daß sie einen Eisengehalt von höchstens 200 ppm besitzt.

12. Kieselsäure nach einem der vorhergehenden Ansprüche, dadurch gekennzeichnet, daß sie einen Calciumgehalt von höchstens 500 ppm, insbesondere von höchstens 300 ppm, besitzt.

13. Kieselsäure nach einem der vorhergehenden Ansprüche, dadurch gekennzeichnet, daß sie einen Kohlenstoffgehalt von höchstens 50 ppm, insbesondere von höchstens 10 ppm, besitzt.

14. Kieselsäure nach einem der vorhergehenden Ansprüche, dadurch gekennzeichnet, daß sie einen pH-Wert von höchstens 8, insbesondere zwischen 6,0 und 7,5, besitzt.

15. Kieselsäure nach einem der vorhergehenden Ansprüche, dadurch gekennzeichnet, daß sie eine BET-Oberfläche zwischen 40 und 600 $m^2/g$ besitzt.

**16.** Kieselsäure nach einem der vorhergehenden Ansprüche, dadurch gekennzeichnet, daß sie eine CTAB-Oberfläche zwischen 40 und 400 $m^2$/g besitzt.

**17.** Kieselsäure nach einem der vorhergehenden Ansprüche vom Schleifmitteltyp, dadurch gekennzeichnet, daß sie eine BET-Oberfläche zwischen 40 und 300 $m^2$/g besitzt.

**18.** Kieselsäure nach Anspruch 17, dadurch gekennzeichnet, daß sie eine CTAB-Oberfläche zwischen 40 und 100 $m^2$/g besitzt.

**19.** Kieselsäure nach einem der Ansprüche 1 bis 16 vom Verdickungsmitteltyp, dadurch gekennzeichnet, daß sie eine BET-Oberfläche zwischen 120 und 450 $m^2$/g, insbesondere 120 und 200 $m^2$/g besitzt.

**20.** Kieselsäure nach Anspruch 19, dadurch gekennzeichnet, daß sie eine CTAB-Oberfläche zwischen 120 und 400 $m^2$/g besitzt.

**21.** Kieselsäure nach einem der Ansprüche 1 bis 16 vom bifunktionellen Typ, dadurch gekennzeichnet, daß sie eine BET-Oberfläche zwischen 80 und 200 $m^2$/g besitzt.

**22.** Kieselsäure nach Anspruch 21, dadurch gekennzeichnet, daß sie eine CTAB-Oberfläche zwischen 80 und 200 $m^2$/g besitzt.

**23.** Kieselsäure nach einem der vorhergehenden Ansprüche, dadurch gekennzeichnet, daß sie ein Ölaufnahmevermögen zwischen 80 und 500 $cm^3$/100 g besitzt.

**24.** Kieselsäure nach einem der vorhergehenden Ansprüche, dadurch gekennzeichnet, daß sie eine mittlere Teilchengröße zwischen 1 und 10 $\mu$m besitzt.

**25.** Kieselsäure nach einem der vorhergehenden Ansprüche, dadurch gekennzeichnet, daß sie eine Schüttdichte zwischen 0,01 und 0,3 besitzt.

**26.** Kieselsäure nach einem der vorhergehenden Ansprüche, dadurch gekennzeichnet, daß gefällte Kieselsäure vorliegt.